(19) Europäisches Patentamt
European Patent Office
Office européen des brevets

(11) EP 2 695 891 B1

(12) # EUROPEAN PATENT SPECIFICATION

(45) Date of publication and mention of the grant of the patent:
**21.02.2018 Bulletin 2018/08**

(21) Application number: **11863081.3**

(22) Date of filing: **27.09.2011**

(51) Int Cl.:
***C07K 14/655*** (2006.01) ***G01N 33/74*** (2006.01)
***A61K 51/08*** (2006.01) ***A61K 38/08*** (2006.01)
***A61K 38/31*** (2006.01)

(86) International application number:
**PCT/RU2011/000739**

(87) International publication number:
**WO 2012/138251 (11.10.2012 Gazette 2012/41)**

(54) **OCTAPEPTIDE, RADIOPHARMACEUTICAL AGENT BASED THEREON AND METHOD FOR DIAGNOSING TUMORS**

OKTAPEPTID, DARAUF BASIERENDES RADIOPHARMAKON UND VERFAHREN ZUR DIAGNOSE VON TUMOREN

OCTAPEPTIDE, RADIOPHARMACEUTIQUE SUR LA BASE DE CELUI-CI ET PROCÉDÉ DE DIAGNOSTIC DE TUMEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.04.2011 RU 2011113388**

(43) Date of publication of application:
**12.02.2014 Bulletin 2014/07**

(73) Proprietor: **Aktsionernoe Obschestvo "Pharm-Sintez"**
**Moskow, 111024 (RU)**

(72) Inventors:
- **NAZARENKO, Anna Borisovna**
  **Moscow 121614 (RU)**
- **RABINOVICH, Eduard Zinovievich**
  **Moscow 105554 (RU)**
- **OVCHINNIKOV, Mikhail Vladimirovich**
  **Moscow 117419 (RU)**

(74) Representative: **Held, Stephan et al**
**Meissner Bolte Patentanwälte**
**Rechtsanwälte Partnerschaft mbB**
**Widenmayerstraße 47**
**80538 München (DE)**

(56) References cited:
**WO-A2-02/24235** **WO-A2-02/24235**
**WO-A2-2004/091490** **RU-C2- 2 160 741**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 695 891 B1

## Description

### Pertinent Art

The invention belongs to the chemical and pharmaceutical industry and concerns radiopharmaceutical agents, both for diagnostic and therapeutic purposes, based on peptide drug delivery carriers.

### Prior Knowledge

Currently the most perspective carriers for radiopharmaceutical agents in oncology are peptides characterized by affinity with tumour cell receptors. Peptide-based radiopharmaceutical agents enhance early diagnostics efficiency and treatment management of a number of tumours.

Historically the first agent for neuroendocrine tumour (NET) diagnosing, $^{111}$In-DTPA-D-Phe$^{1}$-Tyr$^{3}$-octreotide (Octreoscan, Russian analogue is octreotide, $^{111}$In), is produced on a commercial scale and is widely used, despite the disadvantages associated first of all with nuclear and physical properties and $^{111}$In radionuclide price [1]. Herewith octreotide, $^{111}$In possess high diagnostic potential for primary NET and metastases identification in gastroentero-pancreas area and liver, but the potential is far less in case of metastases to lungs, interpleural space and bones.

An important achievement in somatostatine analogue use in nuclear medicine was development of an radiopharmaceutical agent on the basis of octreotide derivatives contained as radionuclide $^{68}$Ga [2] - positive emitter for NET diagnosing using positron emission tomography (PET) obtained using $^{68}$Ge/$^{68}$Ga generator. Nowadays $^{68}$Ga is considered to be one of the most perspective isotopes as due to its nuclear and physical properties and availability it may considerably reduce PET centres dependence on cyclotrons for $^{18}$F, $^{11}$C positive emitters widely used in clinical practice.

There are a number of octapeptides which are structural analogues to octreotide and which contain various chelating groups [3]. Structural formulas of such compounds are presented on Fig. 1.

**Fig. 1. Structural formulas of octreotide analogues with DOTA chelated group**

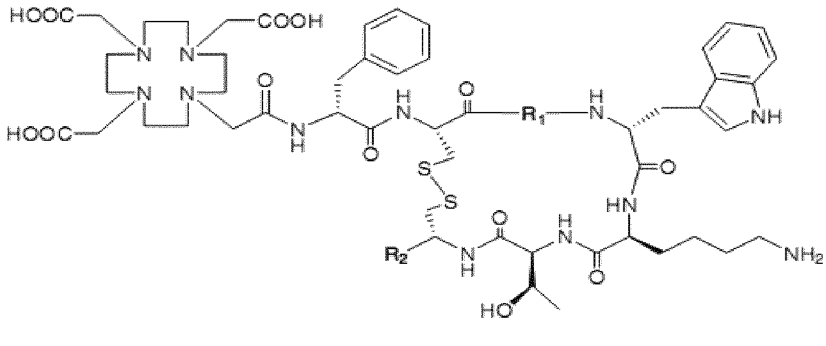

| *Compound* | $R_1$ | $R_2$ |
|---|---|---|
| DOTA-OC | Phe | Thr(ol) |
| DOTA-TOC | Tyr | Thr(ol) |
| DOTA-TATE | Tyr | Thr |
| DOTA-NOC | Nal-1 | Thr(ol) |
| DOTA-NOC-ATE | Nal-1 | Thr |
| DOTA-BOC | BzThi | Thr(ol) |
| DOTA-BOC-ATE | BzThi | Thr |

A number of publications [4-5] were dedicated to studying a range of agents from this group containing chelating group in the form of tetraazacyclododecantetraacetic acid (DOTA) residue and $^{68}$Ga radionuclide. It was demonstrated that these agents are better than octreotide, $^{111}$In in identification of NET and its metastases in lungs, interpleural space and bones. WO 02/24235 A2 discloses DOTA-Tyr3-octreotide complexes with an yttrium label, wherein said compounds

are used to radiolabel tumor tissue.

## Disclosure of the invention

The purpose of this invention is development of new radiopharmaceutical agents with improved imaging properties ensuring enhanced imaging quality for somatostatine receptors expressing tumours.

The engineering purpose is achieved with a new octapeptide being an octreotide derivative for radiopharmaceutical agent development, and also with a radiopharmaceutical agent on the basis thereof and the method to diagnose tumours using this radiopharmaceutical agent.

Therefore, the claimed invention describes a group of inventions comprising a new octapeptide being an octreotide derivative, a radiopharmaceutical agent on the basis thereof and the method to diagnose tumours using this radiopharmaceutical agent.

So, one of the inventions in the claimed group is octapeptide with a common formula (1) to develop radiopharmaceutical agents

X-Lys(Y)-Cys-Tyr-D-Trp-Lys-Thr-Cys-Thr-OH (1)

where X and Y are H, or chelating group capable of forming a complex with the radionuclide and covalently bound to L- or ε- amino groups of N end lysine.

Octapeptide presented within the scope of the invention is distinguished among a number of well-known peptide octreotide analogues with the common formula D-$Phe^1$-$Tyr^3$(octreotide) for the fact that D-$Phe^1$ is replaced with $Lys^1$. Herewith the chelating group may be e.g. DOTA covalently bound to α or ε- amino group of N end lysine in the form of tetraazacyclododecantetraacetic acid (DOTA).

Another invention in the claimed group is a radiopharmaceutical agent being a complex (metal complex) of octapeptide with formula (1) with gallium isotope radionuclide: $^{66}Ga$, $^{67}Ga$, $^{68}Ga$. It allows using the radiopharmaceutical agent with $^{67}Ga$ for SPECT and RNT, and radiopharmaceutical agent with $^{66}Ga$ and $^{68}Ga$ - for PET for tumour and metastases diagnosing.

The engineering task set is achieved also by diagnosing tumours and metastases expressing somatostatine receptors and using the above mentioned radiopharmaceutical agent.

The described compounds - octopeptides with formula (1) being octreotide derivatives may also be used as diagnostic or therapeutic radiopharmaceutical agents in composition with other γ - and β-emitting radionuclides.

As a result of the claimed invention group implementation as an engineering solution, a modern system for early diagnostics of malignant tumours with high somatostatine receptor density will be created (neuroendocrine tumours, central nervous system tumours, breast cancer, small cell carcinoma of lung, etc.). It will allow increasing the number of tumours detected, monitoring therapy dynamics, improving medical services quality and patents' quality of life.

## Best invention implementation method

The claimed invention is illustrated with a number of non-limiting examples.

## Synthesis of octapeptides with common formula 1:

*Abbreviations used:*

DMF - dimethyl formamide
DIPEA - N, N -diisopropylethylamine
HOBT - N-hydroxybenzothiazole
TBTU - tetrafluorborate 2-(1H-benzotriazol-1-yl)-1,1,3,3-tetramethyluronia
TFA - trifluoroacetic acid
Fmoc - fluorenylmethyloxycarbamide
DCC - dicyclohexylcarbodiimide
Trt - trityl
Boc - tert-butyloxycarbonyl
But - tert-butyl

DOTA - tetraazacyclododecantetraacetic acid
DOTA(But)$_3$OH - three-tert-butyl ether of tetraazacyclododecantetraacetic acid
P - polymer.

The synthesis used the protected components from Bachem (Switzerland). Analogies of octapeptides with formula I were synthesised by the solid-phase method.

**Example 1. Synthesis of Fmoc-heptapeptidyl-polymer: Cys(Trt)-Tyr-D-Trp-Lys-Thr-Cys(Trt)-Thr-P**

Note: P means polymer (matrix) used to cultivate Fmoc-heptapeptidyl-polymer and discarded later, namely Wang's polymer by Bachem.

Peptidyl-polymer, the octapeptide predecessor with DOTA chelating group, was synthesised by the solid-phase method using 6.0g of Wang's Fmoc-Thr(But)-polymer by Bachem where the initial amino acid content was 0.61 mmol/g. the condensation reactions were conducted by the dicyclohexylcarbodiimide method using N-hydroxybenzotriazole (DCC/HOBT) in DMF and double derivative surplus: Fmoc-Cys(Trt)-OH, Fmoc-Thr(But)-OH, Fmoc-Lys(Boc)-OH, Fmoc-D-Trp-OH, Fmoc-Tyr(But)-OH. To release amino group protection, 20% piperidine in dimethyl formamide was used. All manipulations were in accordance with the following protocol:

**Table 1.** Synthesis protocol.

| Item No. | Manipulation | No. of treatments (times) | This treatment duration (min) |
|---|---|---|---|
| 1 | Activation of 0.0074 mol of Fmoc amino acid with 0.0074 mol DCC and 0.0074 mol HOBT in 30ml of DMF | 1 | 20 |
| 2 | Peptidyl-polymer flushing with 50ml of DMF prior to protection release | 3 | 1 |
| 3 | Protection release with 30ml of 20% piperidine in DMF | 1 1 | 5 15 |
| 4 | Flushing with 50ml of DMF | 5 | 1 |
| 5 | Condensation | 1 | 120 |
| 6 | Flushing with 50ml of DMF | 3 | 1 |

Upon synthesis completion, peptidyl-polymer was filtered, flushed with DMF (3 × 80ml), methylene chloride (5 × 80ml), and air dried. Resulting 10.36g of Fmoc-heptapeptidyl-polymer was used at later stages.

**Example 2.**

**Synthesis of**

**DOTA-Lys-Cys-Tyr-D-Trp-Lys-Thr-Cys-Thr-OH (1a)**

**meeting the common formula for octapeptide with formula (1)**

**Compound (1a)** was obtained by peptide chain building-up at the last stage according to the protocol given in table 1 using Fmoc-Lys(Boc)-OH at the last synthesis stage. 1.0g (0.5mmol) of protected octapeptidyl-polymer was deprotected with 10ml of 20% DMF piperidine and flushed with DMF according to items (3 and 4) of table 1. Peptidyl-polymer was suspended in 30ml of DMF, then 0.47g (1.0mmol) of respective derivative lysine, 0.15g (1.0mmol) of HOBT, 0.32g (1.0mmol) of TBTU, and 0.5ml of DIPEA were added. The reaction mix was mixed for 2 hours at ambient temperature; the solvent was discarded by filtration; Fmoc-peptidyl-polymer was flushed, deprotected and flushed once again according to items 3 and 4 of table 1. DOTA(But)$_3$OH was coupled using the same reagents which were used to obtain lysine.

Peptidyl-polymer was filtered, flushed with DMF (3 × 30ml), methylene chloride (5 × 30ml), and air dried. 1.1g of peptidyl-polymer was resuspended in 10.0ml of a mix of TFA - tianisole - three-isobutylsylan - ethaneditiol - water

(8.5 : 0.5 : 0.5 : 0.25 : 0.25), and mixed for 4 hours at ambient temperature. The resin was discarded and flushed with TFA (2 x 0.5ml). 50ml of diethyl ether was added to the filtrate; the resulting residue was filtered, flushed with ether (5 ×15ml), ethyl acetate (2 ×15ml). The raw product was dissolved in 0.51 of water, ammonia water solution was added to pH 9, and the mix was left overnight with weak mixing at ambient temperature. 1ml of 5% $H_2O_2$ solution was added to the reaction mix, and mixed for 15 minutes. Disulphide bond completeness was verified using Elman's reagent. The reaction mix was evaporated after adding 1ml of acetic acid (pH 4-5). The product was cleaned by HELC method using diasorb-130 (25×250mm); gradient elution of 0-20% for 10 min and 20-60% for 40 min of buffer solution B in buffer solution A (A - 0.1% TFA and B - 80% acetonitrile in A), flow rate: 12 ml/min, detection at 226nm. Fractions corresponding to the target solution were combined and lyophilized. Compound 1a output is 0.108g, i.e. 15% if recalculating by the initial amino acid, m/z 1416.7 (calculated value: 1416.0), $R_t$ = 13.32 min (analytical HELC using Gilson chromatograph (France), Gromasil C18 column, 4.6 × 250mm, "buffer solution B in buffer solution A" concentration gradient: 20-80% for 30 min where A - 0.1% TFA and B - 80% acetonitrile in A, flow rate: 1 ml/min).

**Example 3.**

**Synthesis**

**H-Lys(DOTA)-Cys-Tyr-D-Trp-Lys-Thr-Cys-Thr-OH (1b),**

**meeting the octapeptide with formula (1)**

Compound 1b was obtained similar to compound 1a; the difference is that Boc-Lys(Fmoc)-OH was used at the last stage of the solid-phase synthesis. (1b) output was 0.099g, i.e. 13% if recalculating by the initial amino acid, m/z 1416.7 (calculated value: 1416.0), $R_t$ = 13.37 min under the conditions similar to those for compound 1a.

Below is an example for obtaining the radiopharmaceutical agent as a complex of the compounds with (1a) and (1b) formulas with a radionuclide, namely **$^{68}$Ga.** Similar radiopharmaceutical agents are obtained using other gallium isotopes - $^{66}$Ga, $^{67}$Ga.

The examples below (4 through 6) illustrate obtaining of the radiopharmaceutical agent (substance) within the scope of the invention and its properties.

**Example 4. Radiopharmaceutical agent on the basis of DOTA-conjugated 1a and 1b peptides marked with $^{68}$Ga**

This method describes the marking reaction with $^{68}$Ga radionuclide of DOTA-conjugated peptides - 1a and 1b octapeptides analogues obtained in examples 2 and 3.

The initial peptide (as a dry matter) is dissolved in deionized water (18 MOm), concentration: 1 ml/ml. The resulting peptide solution is filled with the automatic batcher into 20 $\mu$l polypropylene tubes, type Eppendorf Safe-Lock Tubes (1.5ml).

The packed peptide solution is stored in the freezing chamber at min. - 18°C. Prior to the marking procedure, peptide solution is thawed at ambient temperature for 1-2 min. Acetate buffer is added to the thawed peptide into the same tube and is transferred behind the screen, where $^{68}$Ge/$^{68}$Ga generator eluate (solution of $^{68}$Ga chloride complexes in hydrogen chloride, concentration: 0.1 mol/l) is added to the tube measuring the quantity determined for the peptide used, so that the reaction medium pH is 2.6-3.5 and 3.2-4.2 for 1a and 1b peptides, respectively. The reaction mix is incubated at 80°C for 20 minutes, then the marking process is considered completed.

**Example 5. Radiochemical purity determination for DOTA-conjugated peptides marked with $^{68}$Ga**

The radiochemical purity of the resulting agent was determined by thin layer chromatography (TLC) using two methods.

**Method 1.**

The stationary phase is ITLC-SG strips (Pall Corporation). The eluent is citric acid water solution, concentration: 0.05 mol/l.

$$R_f\ ^{68}{}_{Ga\text{-}free} = 0.8\text{-}1.0$$

$$R_f\ ^{68}{}_{Ga\text{-}bound} = 0.0\text{-}0.2$$

**Method 2.**

The stationary phase is ITLC-SG strips (Pall Corporation). The eluent is the mixture of ammonium acetate water solution (concentration: 1 mol/l) and methanole (1:2).

$$Rf\ ^{68}{}_{Ga\text{-}free} = 0.0\text{-}0.2$$

$$Rf\ ^{68}{}_{Ga\text{-}bound} = 0.8\text{-}1.0$$

When using both methods, the radiochemical purity made 95 ± 4%.

**Example 6. Radiopharmaceutical agent on the basis of 1a and 1b compounds marked with $^{68}$Ga**

The composition of the radiopharmaceutical agent obtained in example 4 is given in table 2.

**Table 2.** Radiopharmaceutical agent on the basis of 1a and 1b compounds marked with $^{68}$Ga

| Item No. | Component | UOM | Compound 1a | Compound 1b |
|---|---|---|---|---|
| 1 | Peptide | mkg | 20 | 20 |
| 2 | Sodium acetate | mg | 8.2-10.6 | 9.4-11.9 |
| 3 | Hydrogen chloride 0,1 M | ml | 1.0 | 1.0 |
| 4 | Potency $^{68}$Ga | mBq | 100-1500 | 100-1500 |

The below given examples (7-8) illustrate the method for tumour diagnosing using the radiopharmaceutical agent within the scope of the invention.

**Example 7. Studying the specificity of the radiopharmaceutical agent on the basis of 1a, 1b compounds marked with $^{68}$Ga, as compared to tumour cells in vitro**

*In **vitro*** experiments used murine melanoma B16 culture from the collection of the Russian Oncology Research Centre named after N.N. Blokhin. The culture used is characterized by the following parameters: morphology - fibroblast-like, viability: 98% (trypan blue colouring on zero passage). The cells were cultivated in culture flasks with the surface area of 25cm$^2$ using the following medium: DMEM medium with FBS added - 10%, L- glutamine - 200 mM, sodium pyruvate - 1 mM, bovine insulin - 10 μkg/ml, and gentamicin (ICN) - 10 U/ml. The cells were incubated at 37°C till full monolayer was obtained, with the cell quantity being 2*10$^6$ cell/vial. 1a and 1b marked compounds and DOTA TATE (DE) comparison substance (0.1ml of each with the peptide concentration of 20μg peptide per 1ml of acetate buffer) were added to the full cell monolayers (2 mln cells per a culture vial with the surface area of 25cm$^2$). DEbased radiopharmaceutical agents are widely used for imaging of tumours expressing somatostatine receptors [6]. DE were marked with $^{68}$Ga by the known method [7]. The cells with radioactive compound were incubated in the thermostat at 37°C for 10, 30 and 60 minutes; then the radioactive solution was discarded, the monolayer was flushed with threefold volume of Hanks' solution. The potency accumulated in melanoma B16 cells was measured by the direct radiometry method in the culture vial. The reference was the same vial containing 5ml of culture medium and the same volume of the agent. Melanoma B16 culture was used in 7 experiments in a separate research, each experimental point was used in a triplet. The results of marked compounds accumulation ($^{68}$Ga-marked compounds (1a), 1b and DE) are given on Figure 1 in percentage of the administered potency normalized for 1 mln cells.

The experiment results show that the investigated compounds bound with tumour cells specifically. It is worth mentioning that the accumulation level for 1a and 1b compounds is higher than in experiments with DE.

**Example 8. Study of accumulation of the radiopharmaceutical agent based on 1a, 1b compounds marked with $^{68}$Ga in animals with tangled tumour expressing somatostatine receptors**

***In vivo*** experiments were conducted using laboratory female mice (F1 CBAxC57B1 hybrids, 18-20g). The animals were obtained from the brooder of the Biomedical Technology Research Centre at the Russian Academy of Medical Sciences in Andreevka. Pigmented B16 mice melanoma was tangled. B16 melanoma cells suspension was administered SC in aseptic conditions. Tumour growth was investigated visually. The animals were enrolled into the experiment 9-12 days after the inoculation, when the tumour diameter was 8-12cm.

During the experiment the animals were housed in standard conditions (special room, recommended food, free access to fresh water, natural lighting).

Solutions of 1a, 1b and DE compounds marked with $^{68}$Ga were administered IV in the caudal vein, in 30 and 60 minutes the animals were decapitated; blood, muscle and tumour tissue as well as main organ (liver, kidney and full urinary bladder) samples were taken. Urinary bladder filling was achieved by suturing the outer urethral orifice. The radioactivity in the sampled organs and tissues was measured by the direct radiometry method. 5 experiments were conducted, at least 3 animals were used for each experimental point. The results are given in table 3.

**Table 3.** Potency distribution in mice bodies with tangled B16 melanoma (% of the administered dose).

| Potency | **DE, marked $^{68}$Ga** | | **1a, marked $^{68}$Ga** | | **1b, marked $^{68}$Ga** | |
|---|---|---|---|---|---|---|
| Organ, tissue | Time after administration, min | | | | | |
| | 30 | 60 | 30 | 60 | 30 | 60 |
| Blood | 2.6 ± 0.5 | 2.6 ± 0.6 | 3.5 ± 0.7 | 1.8 ± 0.5 | 2.4 ± 0.4 | 0.5 ± 0.07 |
| Liver | 2.8 ± 1.5 | 2.8 ± 0.5 | 2.6 ± 0.4 | 3.0 ± 0.6 | 1.8 ± 0.7 | 1.1 ± 0.1 |
| Kidneys | 1.6 ± 0.6 | 1.6 ± 0.6 | 7.6 ± 1.1 | 15.3 ± 2.2 | 3.4 ± 0.3 | 5.4 ± 0.6 |
| Urinary bladder | 21.6 ± 3.2 | 29.2 ± 2.4 | 50.4 ± 4.7 | 72.0 ± 11.9 | 53.4 ± 7.7 | 35.5 ± 3.1 |
| Tumour, %/g | 1.0 ± 0.2 | 0.5 ± 0.24 | 2.6 ± 0.9 | 1.5 ± 0.3 | 2.5 ± 0.7 | 1.2 ± 0.2 |
| Muscle, %/g | 0.6 ± 0.04 | 0.5 ± 0.04 | 1.1 ± 0.5 | 0.2 ± 0.07 | 0.6 ± 0.1 | 0.06 ± 0.01 |
| Tumour/ muscle ratio | 1.7 | 1.0 | 2.4 | **7.5** | 4.2 | **20.0** |

As it is seen from the data obtained, all investigated compounds possess in vivo affinity to melanoma B16 tumour. However the preferred distribution in the body is demonstrated by $^{68}$Ga-marked compounds 1a and 1b as compared to $^{68}$Ga-marked comparison substance DE: faster blood clearance, faster renal clearance, larger 1a and 1b radiopharmaceutical agent accumulation in tumour. Thus, 60 min after administration the 1a and 1b radiopharmaceutical agent accumulation in tumour was 3 and 2.4 times higher as compared to $^{68}$Ga-marked DA. At the same time, the ratio of marked compounds accumulation in tumour and muscle accumulation determining the tumour and metastases imaging quality was higher in 1a and 1b than in DE, both in 30 and in 60 minutes after administration. The largest difference was demonstrated in 60 minutes after administration (**1.0** for DE, **7.5** and **20.0** for 1a and 1b, respectively). This fact is extremely important for PET diagnosing using short-life $^{68}$Ga isotope.

The results confirm considerably better quality of tumour imaging using the radiopharmaceutical agent on the basis of 1a and 1b octapeptides as compared to the DOTA TATE (DE)-based radiopharmaceutical agents.

**Industrial applicability**

The radiopharmaceutical agent is used for diagnosing tumours expressing somatostatine receptors and their metastases.

**Referenced sources:**

1. Q J. Nucl.Med., Mol. Imaging., 49(3):225-35(2005).
2. Q J. Nucl.Med.,46,172S-178S (2005).
3. Eur. J.Nucl. Med. Mol. Imaging., 34,982-993 (2007).
4. Q J Nucl Med. Mol. Imaging., 51: 244-50 (2007).

5. Eur J. Nucl Med Mol Imaging., 34: 1617-1626 (2007).
6. J Nucl Med., 51: 875-882 (2010).
7. J Clin Oncol., 23: 2754-2762 (2005).

## Claims

1. Octapeptide for radiopharmaceutical agents with common formula (1):

X – Lys(Y)-Cys-Tyr-D-Trp-Lys-Thr-Cys-Thr-OH (1),

where X and Y are H, or chelating group, covalently bound to α - or ε- amino groups of N end lysine.

2. Octapeptide according to claim 1 where the chelating group is tetraazacyclododecantetraacetic acid (DOTA).

3. Radiopharmaceutical agent in the form of a complex of compounds with formula (1) with $^{66}Ga$, $^{67}Ga$, $^{68}Ga$ radionuclides.

4. Radiopharmaceutical agent according to claim 3 for use in a method for diagnosing tumours expressing somatostatine receptors or their metastases.

## Patentansprüche

1. Octapeptid für radiopharmazeutische Mittel mit der allgemeinen Formel (1):

X – Lys(Y)-Cys-Tyr-D-Trp-Lys-Thr-Cys-Thr-OH (1),

wobei X und Y H oder Chelatgruppe sind, kovalent gebunden an α- oder ε- Aminogruppen des N terminalen Lysins.

2. Octapeptid nach Anspruch 1, wobei die Chelatgruppe Tetraazacyclododecantetraessigsäure (DOTA) ist.

3. Radiopharmazeutisches Mittel in Form eines Komplexes von Verbindungen mit Formel (1) mit Radionukliden $^{66}Ga$, $^{67}Ga$ , $^{68}Ga$.

4. Radiopharmazeutisches Mittel nach Anspruch 3 zur Verwendung in einem Verfahren zur Diagnose von Tumoren, die Somatostatinrezeptoren exprimieren oder von deren Metastasen.

## Revendications

1. Un octapeptide pour les agents radiopharmaceutiques de la formule générale (1):

X – Lys(Y)-Cys-Tyr-D-Trp-Lys-Thr-Cys-Thr-OH (1),

où X et Y sont H ou le groupe chélatant ayant une liaison covalente avec les groupes aminés α-ou ε- de lysine N-terminale.

2. L'octapeptide selon la revendication 1, où le groupe chélatant est l'acide tétraazacyclododécane tétraacétique

(DOTA).

3. Un agent radiopharmaceutique sous forme d'un complexe des composés de la formule (1) avec les radionucléides $^{66}Ga$, $^{67}Ga$, $^{68}Ga$.

4. L'agent radiopharmaceutique selon la revendication 3 pour la utilisation dans une méthode de diagnostic des tumeurs exprimant des récepteurs de somatostatine ou des leurs métastases.

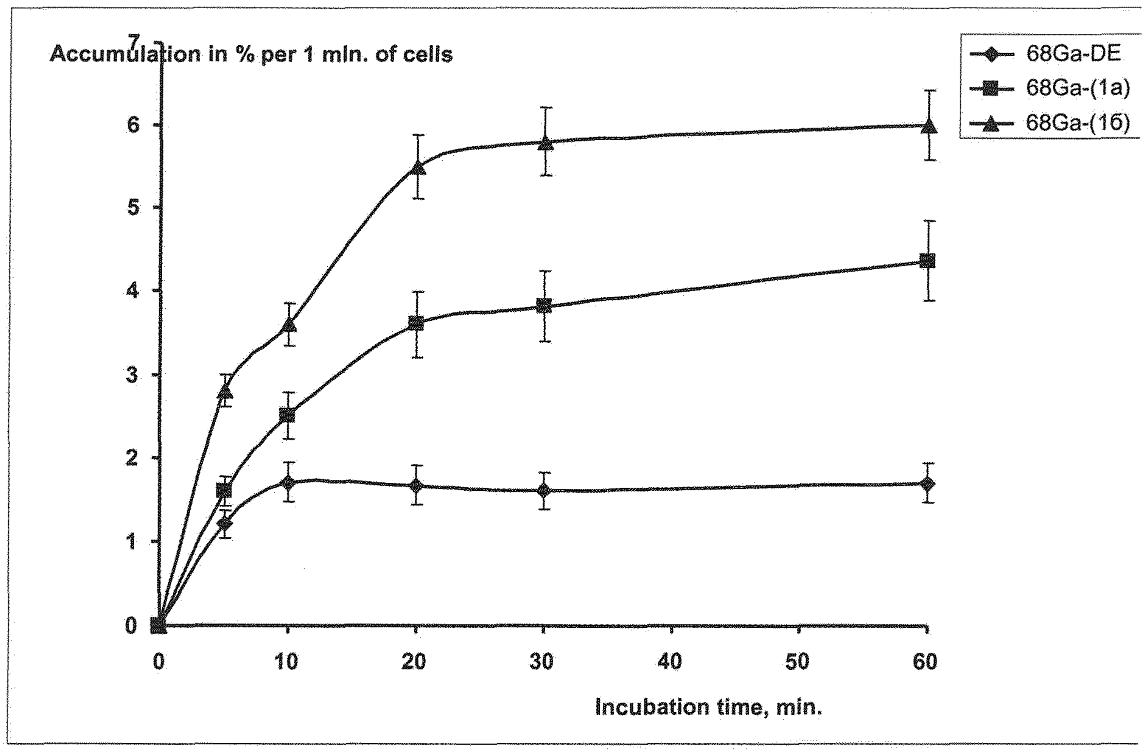


**Fig. 1.** $^{68}$Ga accumulation with marked compounds 1a, 1b and DE.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0224235 A2


### Non-patent literature cited in the description


- Mol. Imaging. *Q J. Nucl.Med.,* 2005, vol. 49 (3), 225-35
- *Q J. Nucl.Med.,* 2005, vol. 46, 172S-178S
- *Eur. J.Nucl. Med. Mol. Imaging.,* 2007, vol. 34, 982-993
- *Q J Nucl Med. Mol. Imaging,* 2007, vol. 51, 244-50
- *Eur J. Nucl Med Mol Imaging,* 2007, vol. 34, 1617-1626
- *J Nucl Med.,* 2010, vol. 51, 875-882
- *J Clin Oncol.,* 2005, vol. 23, 2754-2762